# EUROPEAN PATENT SPECIFICATION

(11) **EP 4 417 285 B1**
(45) Date of publication and mention of the grant of the patent: **01.07.2026**
(21) Application number: 23157085.4
(22) Date of filing: 16.02.2023
(51) Int. Cl.: B01D 15/32, C12N 15/10, A61K 9/51

(54) **METHOD FOR PURIFICATION OF LIPID NANOPARTICLES**
VERFAHREN ZUR REINIGUNG VON LIPIDNANOPARTIKELN
PROCÉDÉ DE PURIFICATION DE NANOPARTICULES LIPIDIQUES

(43) Date of publication of application: 21.08.2024
(73) Proprietor: Sartorius BIA Separations d.o.o., 5270 Ajdovscina (SI)
(72) Inventor: Sprinzar Nemec, Kristina, 5290 Sempeter pri Gorici (SI); Sekirnik, Rok, 1370 Logatec (SI); Raspor, Andrej, 5271 Vipava (SI); Pavlin, Nejc, 5250 Solkan (SI)
(74) Representative: Novagraaf International SA

(56) References cited:
- WO-A1-2015/094928
- WO-A1-2021/158750
- RADU MIHAILA ET AL: "Lipid nanoparticle purification by Spin CentrifugationDialysis (SCD): A facile and high-throughput approach for small scale preparation of siRNAlipid complexes", INTERNATIONAL JOURNAL OF PHARMACEUTICS, ELSEVIER, NL, vol. 420, no. 1, 8 August 2011 (2011-08-08), pages 118 - 121, XP028317625, ISSN: 0378-5173, [retrieved on 20110827], DOI: 10.1016/J.IJPHARM.2011.08.017
- RUYSSCHAERT TRISTAN ET AL: "Liposome retention in size exclusion chromatography", BMC BIOTECHNOLOGY, BIOMED CENTRAL LTD, vol. 5, no. 1, 10 May 2005 (2005-05-10), pages 11, XP021005960, ISSN: 1472-6750, DOI: 10.1186/1472-6750-5-11
- VEMURI S ET AL: "Separation of liposomes by a gel filtration chromatographic technique: a preliminary evaluation", PHARMACEUTICA ACTA HELVETIAE, ELSEVIER BV, NETHERLANDS, vol. 69, no. 2, 1 October 1994 (1994-10-01), pages 107 - 113, XP023817520, ISSN: 0031-6865, [retrieved on 19941001], DOI: 10.1016/0031-6865(94)90009-4

## Description

The present invention relates to methods for the purification of lipid nanoparticles (LNPs) encapsulating a nucleic acid, comprising the steps of subjecting a solution containing said LNPs to a chromatographic medium with convective flow properties in the presence of at least one kosmotropic agent and under hydrophobic interaction chromatography (HIC) conditions; and eluting LNPs from said chromatographic medium. The present invention further relates to respective uses of a chromatographic medium with convective flow properties for the purification of lipid nanoparticles (LNPs) encapsulating a nucleic acid.

LNPs encapsulating nucleic acids have emerged as promising prophylactic and/or therapeutic agents, e.g. as vaccines carrying messenger RNA (mRNA), self-amplifying RNA (saRNA), or circular RNA (circRNA). LNPs are typically composed of ionizable cationic lipids, cholesterol, helper lipids and PEGylated lipids, and have typically a diameter in the range of 30 to 300 nm, e.g. 80 nm. However, LNPs usually are highly shear-sensitive.

The production of e.g. encapsulated mRNA for use as a vaccine consists of multiple unit operations, including microbial plasmid production, plasmid isolation, linearization, *in vitro* transcription reaction to produce mRNA, which is purified by precipitation, chromatography, or tangential flow filtration (TFF) and formulated in low conductivity buffer and mildly acidic conditions. mRNA is then encapsulated into lipid nanoparticles, which are spherical mixtures of mRNA with ionizable lipids, resulting in particles with a diameter of approximately 100 nm. Formulation of mRNA into LNPs is achieved by high pressure in-line mixing in a microfluidic chamber with lipids dissolved in one or more organic solvents, such as ethanol, methanol, acetone, isopropanol, ethyl acetate, or the like.

LNPs thus produced are initially formulated in a high percentage of organic solvent (typically in a range of 10 to 50 % (v/v)), which is detrimental for the stability of nanoparticles and can alter their size. Thus, organic solvents need to be removed from the formulation by means of dilution or buffer exchange. This is commonly achieved by TFF, typically with molecular weight cut-off of 30 kDa to 300 kDa. However, this approach generates high shear forces and turbulences, which lead to the degradation of LNPs, consequently resulting in a low recovery of buffer-exchanged LNPs. In addition, as the cut-off size is smaller than a typical size of mRNA, non-encapsulated mRNA is co-purified and concentrated together with LNPs.

In this context, WO 2021/158750 A1 describes an automated system for lipid exchange-mass spectrometry, e.g., measuring affinity of a membrane protein for lipids, said systems being capable of measuring the specificity of membrane protein-lipid interactions, detecting remodeling of the membrane environment, and determining optimal lipid composition for membrane proteins. Further, Ruysschaert *et al.* (Ruysschaert, T. et al.; BMC Biotechnology, 5(1); May 2005; p. 11) describes methodologies for liposome retention in size exclusion chromatography.

Accordingly, the technical problem underlying the present invention is the provision of methods for the purification of LNPs encapsulating nucleic acids with increased purity and yield.

The solution to the above technical problem is achieved by the embodiments characterized in the claims.

In particular, in a first aspect, the present invention relates to a method for the purification of lipid nanoparticles (LNPs) encapsulating a nucleic acid, comprising the steps of:
(a) subjecting a solution containing said LNPs to a chromatographic medium with convective flow properties in the presence of at least one kosmotropic agent;
(b) washing the chromatographic medium with a solution containing at least one kosmotropic agent; and
(c) eluting LNPs from said chromatographic medium,
wherein step (a) is performed under hydrophobic interaction chromatography (HIC) conditions.

As outlined above, LNPs are nanoparticles that are typically composed of ionizable cationic lipids, cholesterol, helper lipids and PEGylated lipids, typically having a diameter in the range of 30 to 300 nm. However, LNPs that can be purified in the methods of the present invention are not particularly limited and encompass all LNPs that might be of interest for the delivery/administration of nucleic acids.

Likewise, the nucleic acids that are encapsulated in said LNPs are not particularly limited and encompass any nucleic acids that might be of interest in a prophylactic and/or therapeutic setting. Respective nucleic acids encompass single- or double-stranded RNA and DNA molecules of any length, as single species or in combination of two or more species, including e.g. messenger RNA (mRNA), self-amplifying RNA (saRNA), trans-amplifying RNA (taRNA), self-replicating RNA (srRNA), circular RNA (circRNA), guide RNA (gRNA), small interfering RNA (siRNA), and mixtures thereof, wherein gRNA, mRNA, saRNA, and/or circRNA are particularly preferred. The nucleic acids that are encapsulated in said LNPs can also comprise any combinations of two or more encapsulated nucleic acids, e.g. gRNA and mRNA, DNA and mRNA, or the like.

In step (a) of the methods of the present invention, a solution containing the LNPs is subjected to a chromatographic medium having convective flow properties in the presence of at least one kosmotropic agent, wherein said step is performed under hydrophobic interaction chromatography (HIC) conditions.

As used herein, the term "chromatographic medium with convective flow properties" refers to chromatographic media suitable for convective mass transport, i.e., transport of the solution containing the LNPs by convection, e.g. by way of gravity or by way of a pump, and which is not governed by diffusive mass transport. Preferably, the chromatographic medium with convective flow properties has a low hydrophobicity, more preferably a hydrophobicity in the presence of said at least one kosmotropic agent that allows for the binding of the LNPs to the chromatographic medium, but does not allow for the binding of impurities to the chromatographic medium, such as e.g. contaminating nucleic acid potentially present in the solution containing the LNPs. Suitable materials for chromatographic media are not particularly limited and are known in the art. Such materials include synthetic or natural organic polymers. Preferably the chromatographic medium is selected from the group consisting of unmodified or modified styrene-divinyl benzene-based materials, unmodified or modified polymethacrylate-based materials, unmodified or modified cellulose-based materials, and unmodified or modified agarose-based materials. Chromatographic supports can be functionalized with hydroxyl, C4, C6, C8, C12, C18, phenyl or other ligands that exhibit hydrophobic behavior in the presence of kosmotropic salts. Respective chromatographic media can be in the form of chromatographic supports with convective flow properties, such as monoliths, membranes, nano-fibers with pore sizes of at least 0.3 µm and up to 6 µm, preferably of 1 to 2 µm, and porous particles with pore and/or channel sizes of at least 0.3 µm and up to 10 µm, preferably about 1 µm. In preferred embodiments, the chromatographic medium is a monolithic chromatographic medium functionalized with a hydroxyl ligand. Thus, the chromatographic medium can be a monolithic chromatographic medium, a membrane, a nano-fiber, a porous particle as defined above, or any other chromatographic device that exhibits convective mass transport of solutes.

The kosmotropic agent used in step (a) of the methods of the present invention is preferably selected from the group consisting of kosmotropic salts of tetramethyl ammonium, ammonium, potassium, sodium, cesium, lithium, calcium, magnesium, guanidinium, citrates, perchlorates, nitrates, thiocyanates, fluorides, chlorides, sulfates, carbonates, phosphates including pyrophosphates, carboxylates, and combinations thereof. More specifically, the kosmotropic salt can be tetrasodium pyrophosphate (Na₄P₂O₇), monopotassium phosphate (KH₂PO₄), dipotassium phosphate (K₂HPO₄), tripotassium phosphate (K₃PO₄), ammonium sulfate ((NH₄)₂SO₄), sodium chloride (NaCl), monosodium citrate (NaC₆H₇O₇), disodium citrate (Na₂C₆H₆O₇), trisodium citrate (Na₃C₆H₅O₇) or combinations thereof.

The present invention uses a chromatography medium with convective flow properties in the presence of at least one kosmotropic agent, i.e., under specific hydrophobic interaction chromatography (HIC) conditions, wherein these conditions enable the selective binding of the LNPs to the medium. Such HIC conditions include the presence of a kosmotropic agent in a concentration of at least 0.01 M, preferably at least 0.05 M, more preferably of 0.1 to 1 M, more preferably 0.3 to 0.5 M, e.g. about 0.4 M. Moreover, such HIC can further include a conductivity of at least 5 mS/cm and/or a pH in the range of pH 4 to pH 9, preferably pH 5 to pH 8, more preferably about pH 4.6.

In specific embodiments, step (a) of the methods of the present invention is preferably performed at ambient temperature, e.g. at a temperature of between about 20 °C and about 25 °C.

In step (b) of the methods of the present invention, the chromatographic medium is washed with a solution containing at least one kosmotropic agent.

The kosmotropic agent used in step (b) of the methods of the present invention is preferably selected from the group consisting of kosmotropic salts of tetramethyl ammonium, ammonium, potassium, sodium, cesium, lithium, calcium, magnesium, guanidinium, citrates, perchlorates, nitrates, thiocyanates, fluorides, chlorides, sulfates, carbonates, phosphates including pyrophosphates, carboxylates, and combinations thereof. More specifically, the kosmotropic salt can be tetrasodium pyrophosphate (Na₄P₂O₇), monopotassium phosphate (KH₂PO₄), dipotassium phosphate (K₂HPO₄), tripotassium phosphate (K₃PO₄), ammonium sulfate ((NH₄)₂SO₄), sodium chloride (NaCl), monosodium citrate (NaC₆H₇O₇), disodium citrate (Na₂C₆H₆O₇), trisodium citrate (Na₃C₆H₅O₇) or combinations thereof. Preferably, the kosmotropic agent used in step (b) of the methods of the present invention is the same kosmotropic agent use in step (a) of the methods of the present invention.

Further, the solution containing at least one kosmotropic agent used in step (b) of the methods of the present invention preferably comprises the kosmotropic agent in a concentration of at least 0.01 M, preferably at least 0.05 M, more preferably of 0.1 to 1 M, more preferably 0.3 to 0.5 M, e.g. about 0.4 M. Furthermore, said solution can have a conductivity of at least 5 mS/cm and/or a pH in the range of pH 4 to pH 9, preferably pH 5 to pH 8, more preferably about pH 4.6. Preferably, in said solution, the concentration of the kosmotropic agent and/or the conductivity and/or the pH are identical to the concentration of the kosmotropic agent and/or the conductivity and/or the pH used in step (a) of the methods of the present invention.

In specific embodiments, step (b) of the methods of the present invention is preferably performed at ambient temperature, e.g. at a temperature of between about 20 °C and about 25 °C. In this context, said temperature is preferably the same as in step (a) of the methods of the present invention.

In step (c) of the methods of the present invention, the LNPs are eluted from said chromatographic medium, preferably by decreasing the concentration of the kosmotropic agent. Preferably, the eluent used in this step is water, a solution containing no kosmotropic agent or a low concentration of the at least one kosmotropic agent used in step (a), or a buffer with a pH in range of pH 4 to pH 9, e.g. Tris buffer, said buffer containing no kosmotropic agent or a low concentration of the at least one kosmotropic agent. If present at all, such elution might include the presence of a kosmotropic agent in a concentration of at least 0.0001 M, preferably of 0.0005 to 0.002 M, more preferably about 0.001 M.

In specific embodiments, the solution containing LNPs used in the methods of the present invention comprises an organic solvent, e.g. ethanol, derived from the preceding LNP production process. Accordingly, the methods of the present invention can further comprise a step of diluting said solution containing said LNPs in a solution containing said at least one kosmotropic agent prior to step (a). By way of example, the solution containing said LNPs can have an organic solvent concentration typically of between about 10% (v/v) and about 80% (v/v), preferably between about 10% (v/v) and about 50% (v/v), prior to dilution, wherein after dilution, the diluted solution has an organic solvent concentration of 10% (v/v) or less.

In a second aspect, the present invention relates to the use of a chromatographic medium with convective flow properties for the purification of lipid nanoparticles (LNPs) encapsulating a nucleic acid, wherein said chromatographic medium is used in the presence of at least one kosmotropic agent and under hydrophobic interaction chromatography (HIC) conditions.

In this aspect, all relevant limitations as defined for the first aspect of the present invention equally apply to the second aspect of the present invention. In particular, the chromatographic medium with convective flow properties, the LNPs, the nucleic acids and the kosmotropic agents are as defined above.

As used herein, the term "comprising"/"comprises" expressly includes the terms "consisting essentially of"/"consists essentially of" and "consisting of"/"consists of", *i.e.,* all of said terms are interchangeable with each other herein.

Further, as used herein, the term "about" preferably represents a modifier of the specified value of ± 10%, more preferably ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention provides purification methods increasing the yield and purity of LNPs encapsulating nucleic acids, such as mRNA.

Specifically, the present invention provides an approach to concentrate, buffer-exchange and purify LNPs in a single step. Convective chromatographic media, such as monoliths, membranes, and nano-fibers, are composed of interconnected flow-through channels functionalized with ligands. Porous particles with large pores (pore size or channel size larger than 0.3 µm) are also considered as convective chromatographic media. Due to laminar flow properties of mobile phase flowing through interconnected channels or large pores, analytes are exposed to relatively low shear forces compared to TFF. With a suitable choice of ligand, selectivity for the target analyte can be achieved under low shear conditions.

The present invention combines the low shear force environment with ligand chemistry that binds LNP, but not mRNA or other free nucleic acid chains, to achieve concentration, purification and buffer exchange of LNPs after encapsulation.

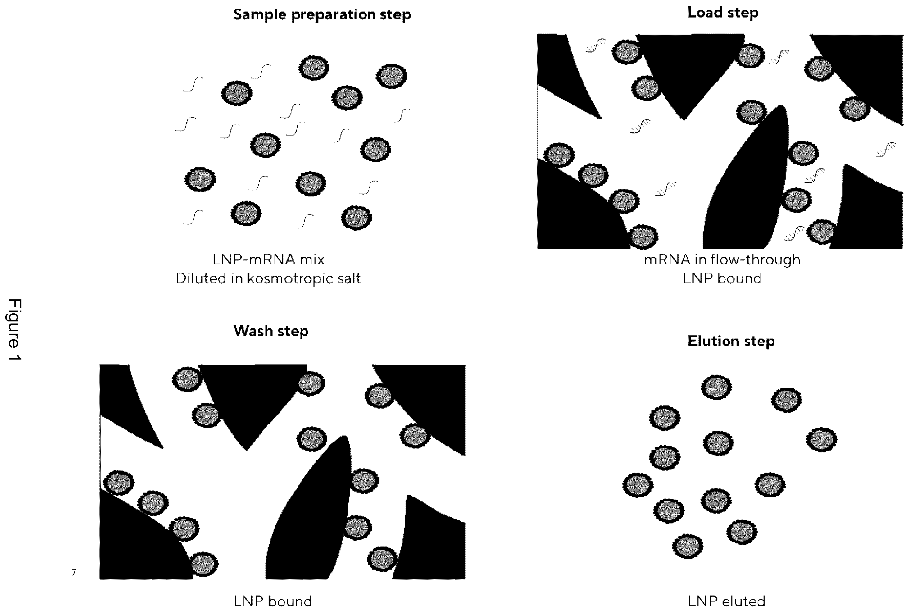

The present invention uses chromatographic media with convective flow properties and with low hydrophobicity under hydrophobic interaction chromatography (HIC) conditions to bind LNPs in the presence of kosmotropic salts, e.g. potassium phosphate, and elute them in low concentration of kosmotropic salt in the presence of buffer, or water, thereby achieving buffer exchange. LNPs formulated in a solution having a high concentration of organic solvent (i.e., the products of the encapsulation step, e.g. having an organic solvent concentration of between about 10% (v/v) and about 80% (v/v)) are first diluted in a mobile phase containing a kosmotropic salt, and applied onto a chromatographic device in the same mobile phase at ambient temperature. Due to convective flow properties of chromatographic media, binding capacity for LNPs is flow-rate independent; consequently, flow rates during loading can be high and loading times short; high initial dilution of LNPs, required to dilute the organic solvent in the mixture, is thus not a limitation. In another embodiment, LNPs formulated in a solution having a high concentration of organic solvent (i.e., the products of the encapsulation step) are first diluted in a low-conductivity matrix (e.g. PBS or Tris buffer), and formulated into loading buffer by in-line dilution immediately prior to loading onto chromatographic medium. As in the previous embodiment, dilution of LNPs can be high without affecting the purification time or efficiency. The hydrophobic matrix of the chromatographic medium (e.g. hydroxyl) retains hydrophobic LNPs during loading. However, due to the lower hydrophobicity of contaminating mRNA or other non-encapsulated nucleic acids compared to LNPs, the former is not retained by the chromatographic unit. LNPs are then eluted in a gradient or step into low conductivity buffer or water (Fig. 1). Due to convective flow properties of the chromatographic medium, elution of LNPs is not governed by diffusion limitations which would lead to chromatographic peak broadening; low volume of eluent (e.g. 3 to 5 column volumes) is required to achieve elution from the chromatographic medium, resulting in high concentration of LNPs in elution fraction, thus concentrating the LNPs.

Applying this feature of chromatographic media with convective flow properties has multiple benefits for the economics and efficiency of downstream purification of LNPs. Firstly, it increases the yield of LNP production compared to methods used in the art, by minimizing exposure of LNPs to destructive shear forces. Secondly, it increases the purification factor compared to methods used in the art by chemical differentiation of target analyte and impurity (e.g. mRNA), not currently achieved by methods used in the art (e.g. TFF, which cannot distinguish between LNP and nucleic acid chain based on molecular weight). This increases purity of the final product, thereby increasing clinical efficacy and decreasing immunogenicity from residual mRNA. Lastly, the methods of the present invention are scalable to industrial (multi-gram) production scales.

The value of this technology could be very high. LNP-encapsulated mRNA or other nucleic acid chains is increasingly used as a stand-alone vaccine for multiple indications. There is a high demand for production of LNPs, with a consequent pressure on increasing the yield of the production/purification process. Current approaches used in the art are suboptimal, as they lead to destruction of particles and do not remove critical impurities. By applying the methods described herein, LNP process yield as well as product purity can be increased, thereby increasing the total process recovery.

The figures show:
Figure 1 :
   A schematic representation of the purification of lipid nanoparticles (LNPs) encapsulating a nucleic acid, with contaminating free nucleic acid species. 1: a sample containing LNP, free (unencapsulated) RNA, residual organic solvent and lipids, is loaded onto a hydrophobic chromatographic medium in the presence of at least one kosmotropic agent; 2) a wash step elutes free RNA, residual organic solvent and lipids from the column while LNPs remain bound; 3) switching of eluent to water (or a solution containing a low concentration of the at least one kosmotropic agent or a buffer with a pH in range of pH 4 to pH 9) releases LNPs from said chromatographic medium.
Figure 2:
   A: Preparative chromatogram of LNP purification with CIMmultus OH monolith (1 mL bed volume, 2 mm channel diameter) with potassium phosphate as kosmotropic salt. Trace A: UV absorbance at 280 nm, trace B: UV absorbance at 260 nm, trace C: conductivity [mS/cm], trace D: multi-angle light scattering (MALS). Load, wash and elution fractions are indicated. B: Zoom-in on loading stage of LNP purification on CIMmultus OH monolith with potassium phosphate as kosmotropic salt. Trace A: UV absorbance at 280 nm, trace B: UV absorbance at 260 nm, trace C: conductivity [mS/cm], trace D: multi-angle light scattering (MALS). An increase in 260 and 280 nm signals is observed, but not MALS, suggesting break-through of free (unencapsulated) mRNA.
Figure 3:
   Analytical reverse-phase (CIMac SDVB) chromatograms of i) mRNA standard, ii) LNP standard, iii) flow-through fraction (FT) from CIMmultus OH purification of LNP preparation, iv) elution fraction (E) from CIMmultus OH purification. Flow-through fraction shows the presence of a species with retention time of ~2.5 min, consistent with elution profile of mRNA; elution fraction shows the presence of a species with retention time of ~6.9 min, consistent with elution profile of LNP.
Figure 4:
   Preparative chromatogram of LNP purification with CIMmultus OH monolith (1 mL bed volume, 2 µm channel diameter) with sodium citrate as kosmotropic salt, as described in Example 2. Trace A: UV absorbance at 260 nm, trace B: multi-angle light scattering (MALS). Load, wash and elution fractions are indicated.
Figure 5:
   Analytical reverse-phase chromatograms of load, flow-through (FT) and elution fraction (E) from CIMmultus OH purification of LNP preparation described in Example 2. Flow-through fraction shows the presence of a species with retention time of ~2.8 min, consistent with elution profile of mRNA; elution fraction shows the presence of a species with retention time of ~7.1 min, consistent with elution profile of LNP.
Figure 6:
   Preparative chromatogram of LNP purification with CIMmultus OH monolith (1 mL bed volume, 2 µm channel diameter) with in-line dilution of LNP into sodium citrate as kosmotropic salt, as described in Example 3. Trace A: UV absorbance at 260 nm, trace B: multi-angle light scattering (MALS). Load, wash and elution fractions are indicated.
Figure 7:
   Analytical reverse-phase chromatograms of load, flow-through (FT) and elution fraction (E) from CIMmultus OH purification of LNP loaded via in-line dilution procedure described in Example 3. Flow-through fraction shows the presence of a species with retention time of ~2.8 min, consistent with elution profile of mRNA; elution fraction shows the presence of a species with retention time of ~7.1 min, consistent with elution profile of LNP.
Figure 8:
   Nanoparticle tracking analysis (NTA) demonstrating particle size distribution of a) load and b) elution fraction of LNP purification with CIMmultus OH monolith with in-line dilution of LNP into sodium citrate as kosmotropic salt, as described in Example 3.

The present invention will be further illustrated by the following examples without being limited thereto.

### Examples

### Example 1:

### Purification of LNPs with CIMmultus OH in potassium phosphate

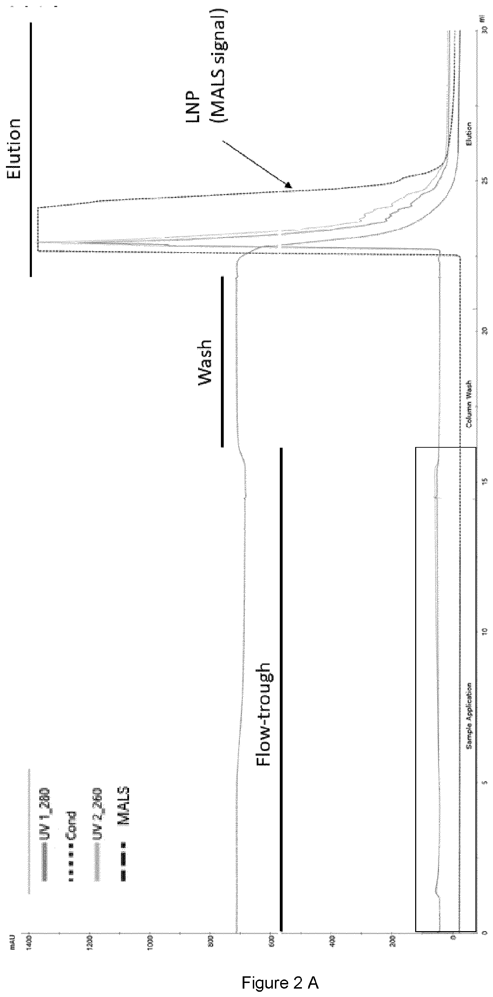

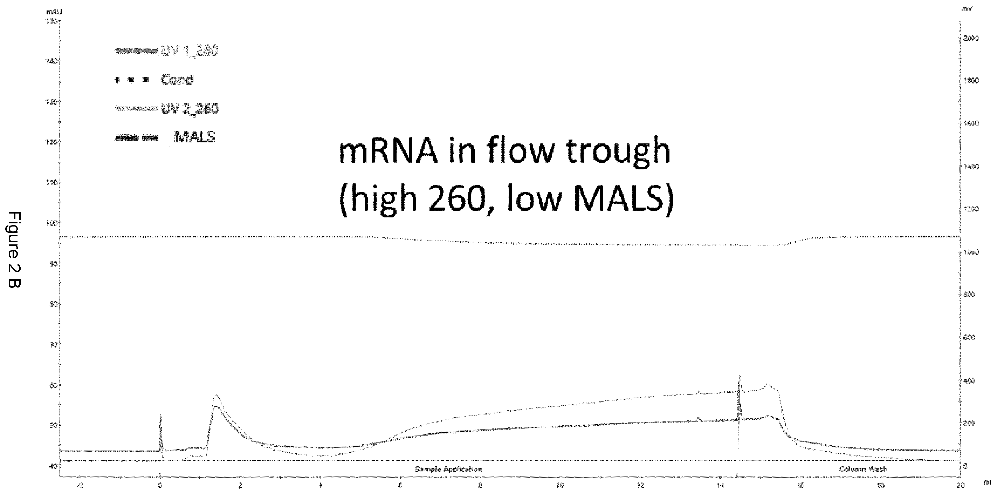

Approximately 100 µg of mRNA (0.2 mg/mL starting concentration) encoding for eGFP (995 nucleotides) was encapsulated into lipid nanoparticles using NanoAssemblr^{®} Ignite^{™} using Genvoy ILM lipid mix according to manufacturer's protocol (N/P ratio 4, total flow-rate 12 mL/min, final sample volume 0.5 mL). LNP sample was directly diluted in 1 M potassium phosphate (total volume 12 mL) and loaded onto CIMmultus OH (1 mL, 2 µm pore size). During sample loading, gradual increase in UV260/280 nm signal, but not MALS signal was observed (Fig. 2B). After sample loading was completed, the column was washed with mobile phase A (1 M potassium phosphate) until UV signal returned to baseline. Elution into 100% ddH₂O was performed in a single step. A large increase in UV260/280 nm and MALS signal was observed.

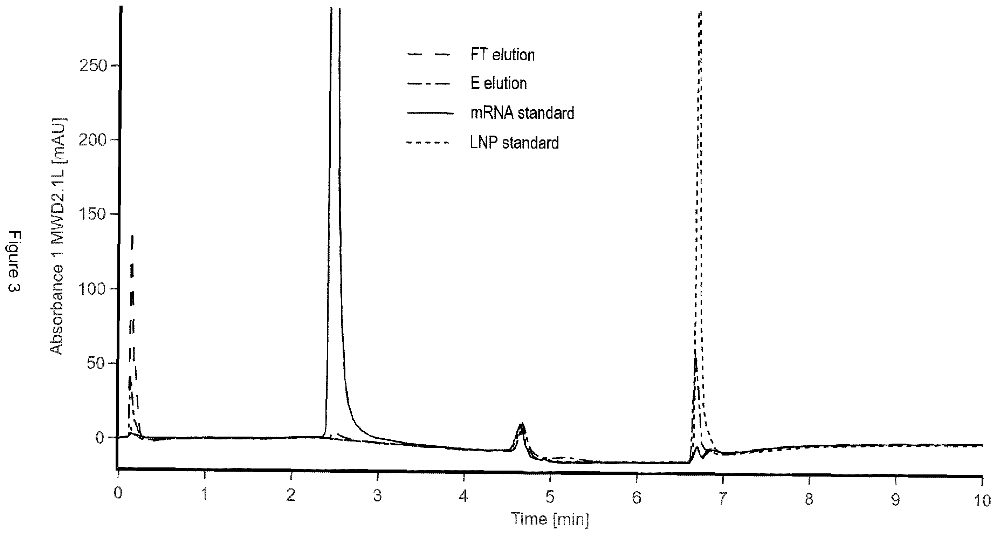

Samples corresponding to flow-through and elution fractions were analyzed by reverse-phase HPLC (Fig. 3) which indicated the presence of RNA, but not LNP, in flow-through and LNP, but not RNA, in elution fraction.

### Example 2:

### Purification of LNPs with CIMmultus OH in sodium citrate

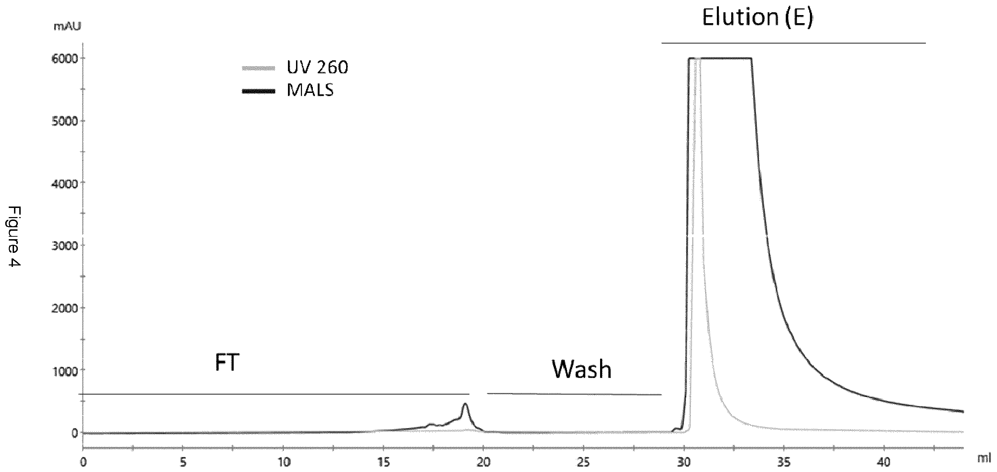

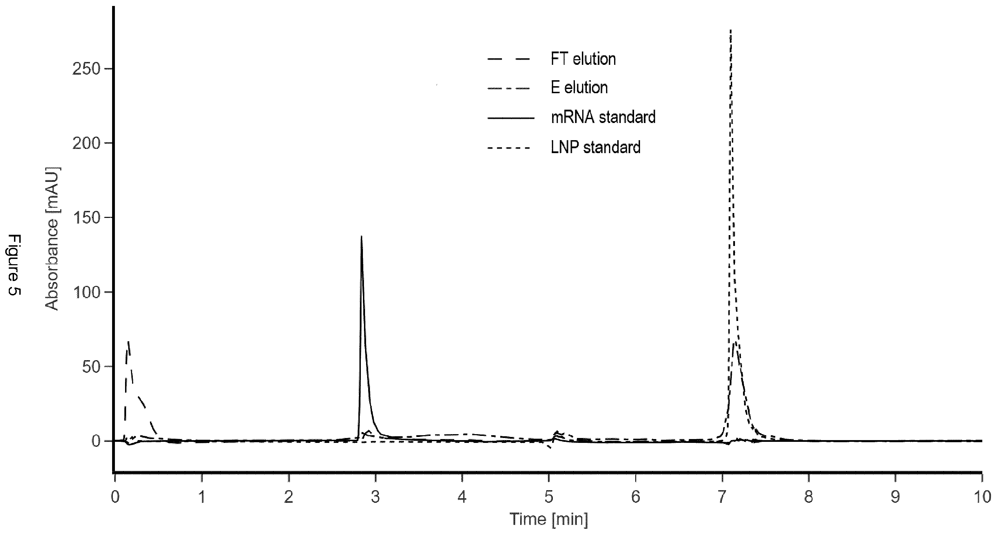

0.2 mg of mRNA encoding for eGFP (995 nucleotides) was encapsulated according to the procedure described above. LNP product (1.6 mL) was immediately diluted 10-fold in PBS to reduce the concentration of ethanol to <3%. 15 mL of this sample was prepared for loading by diluting it in mobile phase A (15 mM Tris, 250 mM Sodium Citrate, pH 7.51) to match MPA conductivity and pH. After sample was loaded, column was washed with 10 mL of MPA. Elution was performed by step from 100% MPA to 100% MPB (15 mM Tris, pH 7.52); 100% MPB was held for 10 mL (10 column volumes). Column strip was performed with deionized water (dH₂O; 10 mL) and column was sanitized with 1 M NaOH (5 column volumes). Chromatographic separation is demonstrated in Figure 4. A small increase in MALS signal at 15 to 20 mL indicated break-through of LNPs (binding capacity exceeded). Strong increases in UV 260 and MALS signals were observed upon elution in MPB (at 30 mL). Reverse-phase chromatographic analysis demonstrated the presence of free (unencapsulated) mRNA in flow-through, but not in elution fraction, as well as presence of LNP in elution fraction (E) and not in flow-through (Fig. 5). Ribogreen analysis (comparison of Ribogreen fluorescence signal of RNA before and after detergent treatment to release encapsulated RNA) of fractions indicated LNP elution recovery of at least 60%. Nanoparticle tracking analysis indicated the presence of particles with diameter of 140-185 nm confirming the presence of LNPs in CIMmultus OH elution.

### Example 3:

### Purification of LNPs with CIMmultus OH in sodium citrate with in-line dilution

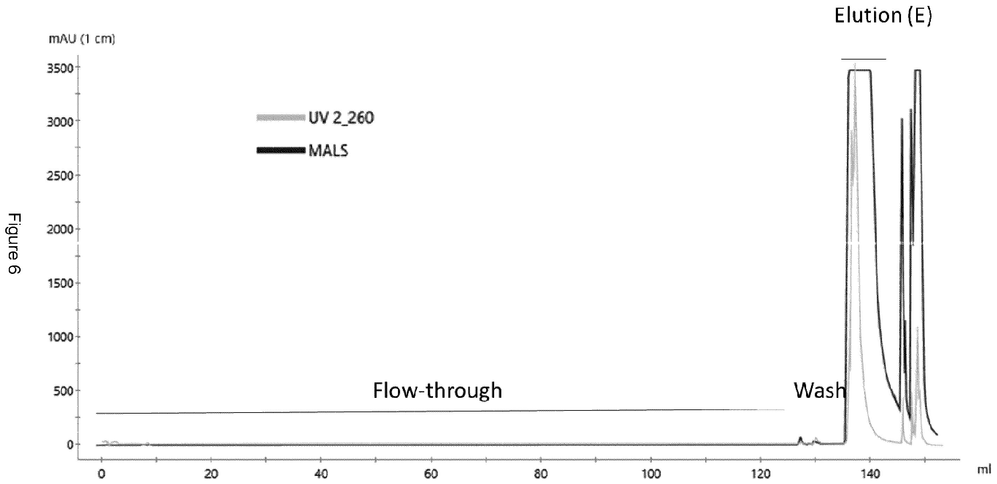

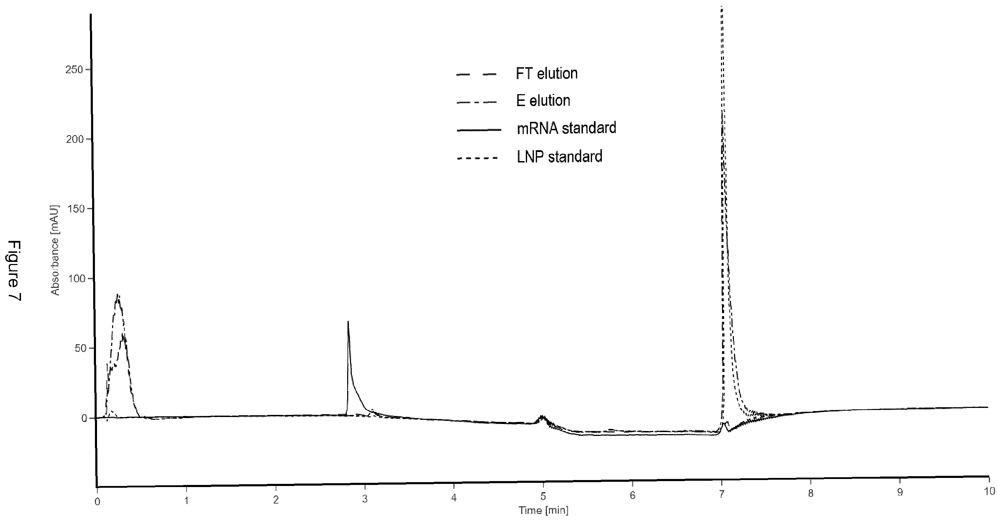

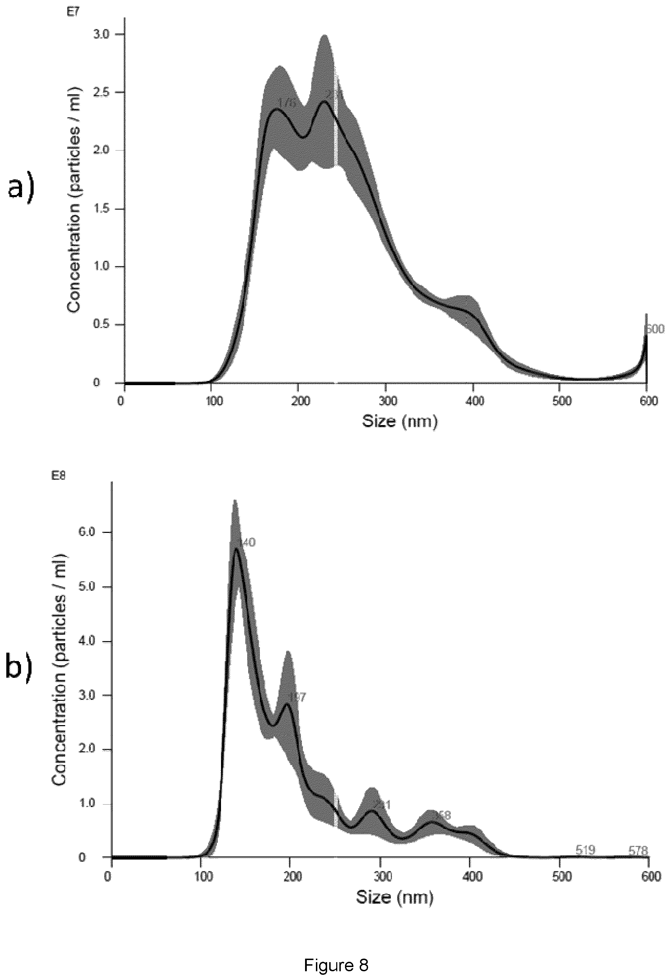

Approximately 0.2 mg of mRNA of 4000 nucleotides (0.1 mg/mL starting concentration) was encapsulated into lipid nanoparticles using NanoAssemblr^{®} Ignite^{™} using Genvoy ILM lipid mix according to manufacturer's protocol (N/P ratio 6, total flow-rate 12 mL/min, final sample volume 1.6 mL). LNP product (1.6 mL) was immediately diluted 10-fold in PBS to reduce the concentration of ethanol to <3%. 15 mL of this sample was loaded by in-line dilution with dilution buffer (30 mM Tris, 500 mM Sodium Citrate, pH 7.5) to match conductivity and pH of MPA (15 mM Tris, 250 mM Sodium Citrate, pH 7.51). After sample was loaded, column was washed with 6 mL of MPA. Elution was performed by step from 100% MPA to 100% MPB (dH₂O); 100% MPB was held for 12 mL (12 column volumes). Column was sanitized with 1 M NaOH (5 column volumes). Chromatographic separation is demonstrated in Figure 6. Strong increases in UV 260 and MALS signals were observed upon step in 100% MPB (Elution), suggesting elution of LNPs. Reverse-phase chromatographic analysis demonstrated a low signal corresponding to free (unencapsulated) mRNA in flow-through, but not in elution fraction, as well as presence of LNP in elution fraction (E) and not in flow-through (FT) (Fig. 7). Ribogreen analysis (comparison of Ribogreen fluorescence signal of RNA before and after detergent treatment to release encapsulated RNA) of fractions indicated LNP elution recovery of 95%. Nanoparticle tracking analysis of load indicated the presence of a wide particle size distribution with mode value of 230 nm, concentration in load was 4.4×10⁹ particles/mL (Fig. 8 a). Elution fraction (E) indicated the presence of particles with diameter of mode value of 142 nm (Fig. 8 b); concentration of particles was 4.5×10¹⁰ particles/mL, confirming the presence of LNPs and demonstrating a 10-fold concentration of particles relative to the load.

## Claims

1. A method for the purification of lipid nanoparticles (LNPs) encapsulating a nucleic acid, comprising the steps of:
(a) subjecting a solution containing said LNPs to a chromatographic medium with convective flow properties in the presence of at least one kosmotropic agent;
(b) washing the chromatographic medium with a solution containing at least one kosmotropic agent; and
(c) eluting LNPs from said chromatographic medium,
wherein step (a) is performed under hydrophobic interaction chromatography (HIC) conditions.

2. The method of claim 1, wherein the chromatographic medium exhibits a hydrophobicity in the presence of said at least one kosmotropic agent that allows for the binding of the LNPs to the chromatographic medium but does not allow for the binding of contaminating nucleic acid to the chromatographic medium.

3. The method of claim 1 or claim 2, wherein the chromatographic medium is a synthetic or natural organic polymer.

4. The method of any one of claims 1 to 3, wherein the chromatographic medium is selected from the group consisting of unmodified or modified styrene-divinyl benzene-based materials, unmodified or modified polymethacrylate-based materials, unmodified or modified cellulose-based materials, and unmodified or modified agarose-based materials.

5. The method of any one of claims 1 to 4, wherein the chromatographic medium is functionalized with hydroxyl, C4, C6, C8, C12, C18, or phenyl ligands.

6. The method of any one of claims 1 to 5, wherein the chromatographic medium is a monolithic chromatographic medium, a membrane, a nano-fiber, or a porous particle.

7. The method of any one of claims 1 to 6, wherein the at least one kosmotropic agent is present in said solution in a concentration of at least 0.01 M.

8. The method of any one of claims 1 to 7, wherein the kosmotropic agent is selected from the group consisting of kosmotropic salts of tetramethyl ammonium, ammonium, potassium, sodium, cesium, lithium, calcium, magnesium, guanidinium, citrates, perchlorates, nitrates, thiocyanates, fluorides, chlorides, sulfates, carbonates, phosphates including pyrophosphates, carboxylates, and combinations thereof.

9. The method of any one of claims 1 to 8, wherein the kosmotropic salt is tetrasodium pyrophosphate (Na₄P₂O₇), monopotassium phosphate (KH₂PO₄), dipotassium phosphate (K₂HPO₄), tripotassium phosphate (K₃PO₄), ammonium sulfate ((NH₄)₂SO₄), sodium chloride (NaCl), monosodium citrate (NaC₆H₇O₇), disodium citrate (Na₂C₆H₆O₇), trisodium citrate (Na₃C₆H₅O₇), or combinations thereof.

10. The method of any one of claims 1 to 9, wherein HIC conditions include the presence of a kosmotropic agent in a concentration of at least 0.01 M, and/or a conductivity of at least 5 mS/cm, and/or a pH in the range of pH 4 to pH 9.

11. The method of any one of claims 1 to 10, wherein the eluent used in step (c) is water, a solution containing no kosmotropic agent or a low concentration of the at least one kosmotropic agent used in step (a), or a buffer with a pH in range of pH 4 to pH 9, said buffer containing no kosmotropic agent or a low concentration of the at least one kosmotropic agent.

12. The method of any one of claims 1 to 11, wherein the solution containing said LNPs comprises one or more organic solvents in a concentration of between about 10% (v/v) and about 80% (v/v).

13. The method of any one of claims 1 to 12, wherein the nucleic acid is selected from the group consisting of messenger RNA (mRNA), self-amplifying RNA (saRNA), trans-amplifying RNA (taRNA), self-replicating RNA (srRNA), circular RNA (circRNA), guide RNA (gRNA), small interfering RNA (siRNA), and mixtures thereof.

14. Use of a chromatographic medium with convective flow properties for the purification of lipid nanoparticles (LNPs) encapsulating a nucleic acid, wherein said chromatographic medium is used in the presence of at least one kosmotropic agent and under hydrophobic interaction chromatography (HIC) conditions.

## Patentansprüche

1. Verfahren zur Reinigung von Lipidnanopartikeln (LNP), die eine Nukleinsäure enkapsulieren, umfassend die folgenden Schritte:
(a) Unterziehen einer Lösung, die die genannten LNP enthält, einem chromatografischen medium mit Konvektionsströmungseigenschaften in Gegenwart von mindestens einem kosmotropen Agens ;
(b) Waschen des chromatografischen Mediums mit einer Lösung, die mindestens ein kosmotropes Agens enthält ; und
(c) Eluieren der LNP von dem genannten chromatografischen Medium,
wobei Schritt (a) unter Bedingungen der hydrophoben Interaktionschromatographie (HIC) durchgeführt wird.

2. Verfahren nach Anspruch 1, wobei das chromatografische Medium in Gegenwart des genannten mindestens einen kosmotropen Agens eine Hydrophobizität aufweist, die die Bindung der LNP an das chromatografische Medium erlaubt, jedoch nicht die Bindung kontaminierender Nukleinsäuren an das chromatografische Medium erlaubt.

3. Verfahren nach Anspruch 1 oder Anspruch 2, wobei das chromatografische Medium ein synthetisches oder natürliches organisches Polymer ist.

4. Verfahren nach einem der Ansprüche 1 bis 3, wobei das chromatografische Medium ausgewählt ist aus der Gruppe bestehend aus unmodifizierten oder modifizierten Styrol-Divinylbenzol-basierten Materialien, unmodifizierten oder modifizierten Polymethacrylatbasierten Materialien, unmodifizierten oder modifizierten Zellulose-basierten Materialien, und unmodifizierten oder modifizierten Agarose-basierten Materialien.

5. Verfahren nach einem der Ansprüche 1 bis 4, wobei das chromatografische Medium mit Hydroxyl-, C4-, C6-, C8-, C12-, C18- oder Phenyl-Liganden funktionalisiert ist.

6. Verfahren nach einem der Ansprüche 1 bis 5, wobei das chromatografische Medium ein monolithisches chromatografisches Medium, eine Membran, eine Nanofaser oder ein poröses Partikel ist.

7. Verfahren nach einem der Ansprüche 1 bis 6, wobei das mindestens eine kosmotrope Agens in der genannten Lösung in einer Konzentration von mindestens 0,01 M vorhanden ist.

8. Verfahren nach einem der Ansprüche 1 bis 7, wobei das kosmotrope Agens ausgewählt ist aus der Gruppe bestehend aus kosmotropen Salzen von Tetramethylammonium, Ammonium, Kalium, Natrium, Cäsium, Lithium, Kalzium, Magnesium, Guanidinium, Citraten, Perchloraten, Nitraten, Thiocyanaten, Fluoriden, Chloriden, Sulfaten, Karbonaten, Phosphaten einschließlich Pyrophosphaten, Carboxylaten, und Kombinationen davon.

9. Verfahren nach einem der Ansprüche 1 bis 8, wobei das kosmotrope Salz Natriumpyrophosphat (Na₄P₂O₇), Monokaliumphosphat (KH₂PO₄), Dikaliumphosphat (K₂HPO₄), Trikaliumphosphat (K₃PO₄), Ammoniumsulfat ((NH₄)₂SO₄), Natriumchlorid (NaCl), Mononatriumcitrat (NaC₆H₇O₇), Dinatriumcitrat (Na₂C₆H₆O₂), Trinatriumcitrat (Na₃C₆H₅O₇) oder Kombinationen davon ist.

10. Verfahren nach einem der Ansprüche 1 bis 9, wobei die HIC-Bedingungen die Anwesenheit eines kosmotropen Agens in einer Konzentration von mindestens 0,01 M, und/oder eine Leitfähigkeit von mindestens 5 mS/cm, und/oder einen pH-Wert im Bereich von pH 4 bis pH 9 umfassen.

11. Verfahren nach einem der Ansprüche 1 bis 10, wobei das in Schritt (c) verwendete Elutionsmittel Wasser, eine Lösung, die kein kosmotropes Agens oder eine niedrige Konzentration des mindestens einen in Schritt (a) verwendeten kosmotropen Agens enthält, oder ein Puffer mit einem pH-Wert im Bereich von pH 4 bis pH 9, wobei der genannte Puffer kein kosmotropes Agens oder eine niedrige Konzentration des mindestens einen kosmotropen Agens enthält, ist.

12. Verfahren nach einem der Ansprüche 1 bis 11, wobei die die genannten LNP enthaltende Lösung ein oder mehrere organische Lösungsmittel in einer Konzentration von etwa 10 % (v/v) bis etwa 80 % (v/v) umfasst.

13. Verfahren nach einem der Ansprüche 1 bis 12, wobei die Nukleinsäure ausgewählt ist aus der Gruppe bestehend aus Boten-RNA (mRNA), selbst-amplifizierender RNA (saRNA), trans-amplifizierender RNA (taRNA), selbst-replizierender RNA (srRNA), zirkulärer RNA (circRNA), Leit-RNA (gRNA), kleiner interferierender RNA (siRNA), und deren Gemischen.

14. Verwendung eines chromatografischen Mediums mit Konvektionsströmungseigenschaften zur Reinigung von Lipidnanopartikeln (LNP), die eine Nukleinsäure enkapsulieren, wobei das genannte chromatografische Medium in Gegenwart von mindestens einem kosmotropen Agens und unter Bedingungen der hydrophoben Interaktionschromatographie (HIC) verwendet wird.

## Revendications

1. Procédé de purification de nanoparticules lipidiques (LNP) encapsulant un acide nucléique, comprenant les étapes :
(a) de soumission d'une solution contenant lesdites LNP à un milieu chromatographique ayant des propriétés d'écoulement convectif en présence d'au moins un agent kosmotropique ;
(b) de lavage du milieu chromatographique avec une solution contenant au moins un agent kosmotropique ; et
(c) d'élution des LNP dudit milieu chromatographique,
l'étape (a) étant réalisée dans des conditions de chromatographie d'interaction hydrophobe (HIC).

2. Procédé selon la revendication 1, dans lequel le milieu chromatographique présente une hydrophobicité en présence dudit au moins un agent kosmotropique qui permet la fixation des LNP sur le milieu chromatographique mais ne permet pas la fixation d'acides nucléiques contaminants sur le milieu chromatographique.

3. Procédé selon la revendication 1 ou la revendication 2, dans lequel le milieu chromatographique est un polymère organique synthétique ou naturel.

4. Procédé selon l'une quelconque des revendications 1 à 3, dans lequel le milieu chromatographique est sélectionné dans le groupe constitué de matériaux à base de styrène-divinylbenzène non modifiés ou modifiés, de matériaux à base de polyméthacrylate non modifiés ou modifiés, de matériaux à base de cellulose non modifiés ou modifiés, et de matériaux à base d'agarose non modifiés ou modifiés.

5. Procédé selon l'une quelconque des revendications 1 à 4, dans lequel le milieu chromatographique est fonctionnalisé avec des ligands hydroxyle, C4, C6, C8, C12, C18 ou phényle.

6. Procédé selon l'une quelconque des revendications 1 à 5, dans lequel le milieu chromatographique est un milieu chromatographique monolithique, une membrane, une nano-fibre ou une particule poreuse.

7. Procédé selon l'une quelconque des revendications 1 à 6, dans lequel l'au moins un agent kosmotropique est présent dans ladite solution à une concentration d'au moins 0,01 M.

8. Procédé selon l'une quelconque des revendications 1 à 7, dans lequel l'agent kosmotropique est sélectionné dans le groupe constitué de sels kosmotropiques d'ammonium tétraméthyle, d'ammonium, de potassium, de sodium, de césium, de lithium, de calcium, de magnésium, de guanidinium, de citrates, de perchlorates, de nitrates, de thiocyanates, de fluorures, de chlorures, de sulfates, de carbonates, de phosphates y compris les pyrophosphates, de carboxylates, et leurs combinaisons.

9. Procédé selon l'une quelconque des revendications 1 à 8, dans lequel le sel kosmotropique est le pyrophosphate de tétrasodium (Na₄P₂O₇), le phosphate de monopotassium (KH₂PO₄), le phosphate de dipotassium (K₂HPO₄), le phosphate de tripotassium (K₃PO₄), le sulfate d'ammonium ((NH₄)₂SO₄), le chlorure de sodium (NaCl), le citrate de monosodium (NaC₆H₇O₇), le citrate de disodium (Na₂C₆H₆O₇), le citrate de trisodium (Na₃C₆H₅O₇), ou leurs combinaisons.

10. Procédé selon l'une quelconque des revendications 1 à 9, dans lequel les conditions HIC comprennent la présence d'un agent kosmotropique à une concentration d'au moins 0,01 M, et/ou une conductivité d'au moins 5 mS/cm, et/ou un pH dans la plage de pH 4 à pH 9.

11. Procédé selon l'une quelconque des revendications 1 à 10, dans lequel l'éluant utilisé à l'étape (c) est de l'eau, une solution ne contenant pas d'agent kosmotropique ou une faible concentration de l'au moins un agent kosmotropique utilisé à l'étape (a), ou un tampon avec un pH dans la plage de pH 4 à pH 9, ledit tampon ne contenant pas d'agent kosmotropique ou une faible concentration de l'au moins un agent kosmotropique.

12. Procédé selon l'une quelconque des revendications 1 à 11, dans lequel la solution contenant lesdites LNP comprend un ou plusieurs solvants organiques à une concentration d'environ 10 % (v/v) à environ 80 % (v/v).

13. Procédé selon l'une quelconque des revendications 1 à 12, dans lequel l'acide nucléique est sélectionné dans le groupe constitué de l'ARN messager (ARNm), de l'ARN auto-amplifiant (saRNA), de l'ARN trans-amplifiant (taRNA), de l'ARN autoréplicatif (srRNA), de l'ARN circulaire (circRNA), de l'ARN guide (ARNg), de l'ARN interférent de petite taille (ARNsi), et leurs mélanges.

14. Utilisation d'un milieu chromatographique ayant des propriétés d'écoulement convectif pour la purification de nanoparticules lipidiques (LNP) encapsulant un acide nucléique, dans laquelle ledit milieu chromatographique est utilisé en présence d'au moins un agent kosmotropique et dans des conditions de chromatographie d'interaction hydrophobe (HIC).
